# EUROPEAN PATENT APPLICATION

(11) **EP 0 739 894 A1**
(43) Date of publication of application: **30.10.1996**
(21) Application number: 95905755.5
(22) Date of filing: 11.01.1995
(51) Int. Cl.: C07D 417/12, A61K 31/44

(54) **THIAZOLINE DERIVATIVE**

(30) Priority: 14.01.1994 JP 2722/94
(71) Applicant: TAISHO PHARMACEUTICAL CO. LTD, Tokyo 171 (JP)
(72) Inventor: SATO, Masakazu Taisho Pharmaceutical Co., Ltd., Toshima-ku Tokyo 171 (JP); MANAKA, Akira Taisho Pharmaceutical Co., Ltd., Toshima-ku Tokyo 171 (JP); TAKAHASHI, Keiko Taisho Pharmaceutical Co., Ltd., Toshima-ku Tokyo 171 (JP); KAWASHIMA, Yutaka Taisho Pharmaceutical Co., Ltd., Toshima-ku Tokyo 171 (JP); HATAYAMA, Katsuo Taisho Pharmaceutical Co., Ltd., Toshima-ku Tokyo 171 (JP)
(74) Representative: Weisert, Annekäte, Dipl.-Ing. Dr.-Ing.
(86) International application number: JP9500018
(87) International publication number: WO9519360

(57) **Abstract**

OBJECT: To provide compounds having an excellent inhibitory action of blood platelet aggregation and a good stability when formulated.

CONSTITUTION: A thiazoline derivative represented by the formula: 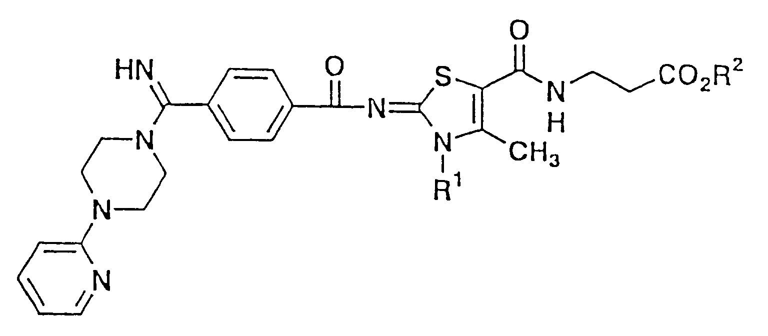 wherein R¹ is an alkyl group having 1 to 4 carbon atoms and R² is a hydrogen atom or an alkyl group having 1 to 4 carbon atoms, or a pharmaceutically acceptable salt thereof.

## Description

### TECHNICAL FIELD

The present invention relates to novel thiazoline derivatives having an excellent inhibitory action of blood platelet aggregation.

### BACKGROUND ART

Compounds of the present invention are included within the Formula (I) described in the claim of WO 94/02472, but not specifically described in the specification thereof. The compounds specifically described in the specification of WO 94/02472 have an excellent inhibitory action of blood platelet aggregation, but have a drawback such as a poor solubility when formulated, especially formulated into the form of injectional solutions. An object of the present invention is to provide compounds having an excellent inhibitory action of blood platelet aggregation, a good solubility when formulated, especially formulated into the form of injectional solutions, and good stability in the above preparations with time, e.g. compounds for pratical use as medicines.

### DISCLOSURE OF THE INVENTION

As a result of extensive reseaches, the present inventors have found that certain thiazoline derivatives have an excellent effect which is unexpected from that of the compounds specifically described in the specification of WO 94/02472 and achieve the above-mentioned object, and thereby the present invention has been accomplished.

Namely, the present invention is a thiazoline derivative represented by the formula: 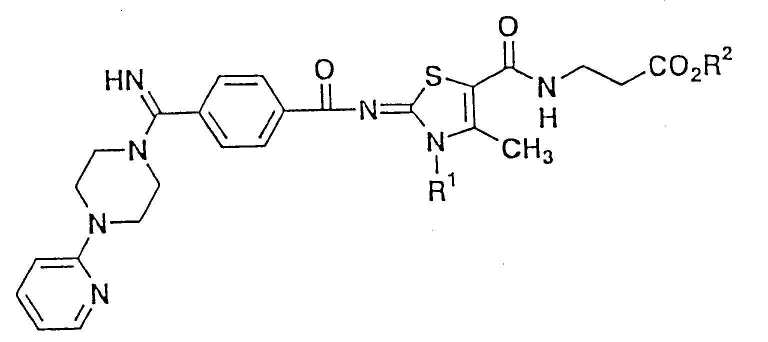 wherein R¹ is an alkyl group having 1 to 4 carbon atoms and R² is a hydrogen atom or an alkyl group having 1 to 4 carbon atoms, or a pharmaceutically acceptable salt thereof.

In the present invention, the alkyl group having 1 to 4 carbon atoms refers to a methyl group, an ethyl group, an n-propyl group, an isopropyl group, an n-butyl group, an isobutyl group, a sec-butyl group or a t-butyl group. The pharmaceutically acceptable salt of the compound of Formula (I), not limited thereto, refers to any pharmaceutically acceptable salts, for example, salts with alkali metals, alkali earth metals, ammonia, alkylamines, mineral acids, carboxylic acids or sulfonic acids, and especially sodium salt, potassium salt, calcium salt, ammonium salt, aluminium salt, triethylammonium salt, 1-adamantylamine salt, hydrochloride, hydrobromide, hydroiodide, sulfate, nitrate, phosphate, acetate, fumarate, malate, citrate, methanesulfonate or p-toluenesulfonate.

The compounds of the present invention can be prepared, for example, by the following methods.

Namely, a compound represented by the formula: 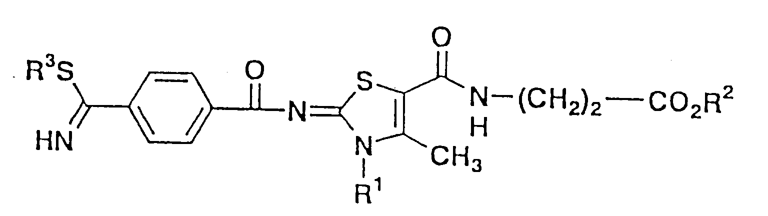 (wherein R³ is a lower alkyl group, and R¹ and R² are as defined above) or a salt thereof obtained by the method described on page 4 of the specification of WO 94/02472 is reacted with 1-(pyridin-2-yl)piperazine or a salt thereof to give a compound of Formula (I) or a pharmaceutically acceptable salt thereof.

A compound of Formula (I) wherein R² is a hydrogen atom or a pharmaceutically acceptable salt thereof can be also prepared by hydrolysis of the ester moiety of the compound of Formula (I) wherein R² is an alkyl group having 1 to 4 carbon atoms or a pharmaceutically acceptable salt thereof. The above-mentioned hydrolysis to be used is an ordinary method such as treatments with an alkali, a mineral acid or an organic acid.

In the above reaction, a base can be used, examples of which are alkali earth metal salts (e.g. sodium hydroxide, potassium hydroxide, potassium acetate, sodium acetate, sodium dimsyl, sodium hydride, calcium hydride, sodium amide or potassium tert-butyl) and amines (e.g. triethylamine, diisopropylethylamine or pyridine). The salt of 1-(pyridin-2-yl)piperazine to be used includes organic acid salts such as acetate. A reaction solvent to be used includes, for example, a reaction-inert solvent such as alcohols (e.g. methanol, ethanol, isopropyl alcohol or tert-butyl alcohol), N,N-dimethylformamide, dimethyl sulfoxide, pyridine, methylene chloride, chloroform, acetone or acetic acid.

### INDUSTRIAL UTILIZATION

The thus-obtained compounds of the present invention exert an excellent inhibitory action of blood platelet aggregation and are stable with time after formulating, especially after forming into injectional solutions.

Accordingly, the compounds of the present invention are useful as preventive and therapeutic agents for ischemic diseases (e.g. thrombosis, cerebral infarction or myocardial infarction) and arteriosclerosis diseases.

For the purposes, the compounds of the present invention are mixed with, for example, conventional fillers, binders, disintegrators, pH moderators or solubilizers, and prepared in the forms such as tablets, pills, capsules, granules, powders, solutions, emulsions, suspensions or injectional solutions, and preferably injectional solutions, by conventional formulation techniques.

The dose for adult patients is 1 to 1000 mg for oral route, and 0.01 to 100 mg for parenteral route, which can be administered in a single dose or in several divided doses per day. This dose can be increased or decreased depending on the kind of the diseases and the age, body weight and conditions of the patient.

The excellent action of the compounds of the present invention is illustrated by the following experiments.

### Experiment 1 [Human in vitro Blood Platelet Aggregation Inhibition Test]

Citrated blood (the volume ratio of 3.13 % sodium citrate solution and blood is 1:9) was collected from the cubital vein of a healthy human who had not received any drugs known to affect the function of blood platelet within 2 weeks prior to starting the test, and centrifuged at 120 x g at room temperature for 15 minutes. The resulting supernatant was separated to give platelet rich plasma (PRP), the remaining precipitate was further centrifuged at 1500 x g for 10 minutes, and the resulting supernatant was separated to give platelet poor plasma (PPP).

The blood platelet counts of PRP were adjusted to 50 ∼ 60 × 10⁴/µl by diluting with PPP.

Blood platelet aggregation was determined according to the method of Born G.V.R., [Nature, vol. 194, page 927 (1962)] using adenosine diphosphate (produced by Sigma Co.; hereinafter referred to as "ADP") as an aggregation-inducing substance. That is, a solution of the compound of Formula (I) as a test drug in dimethyl sulfoxide was adjusted to the desired concentration with a physiological saline solution. 25 µl of the solution was added to 250 µl of PRP and incubated at 37°C for 3 minutes, and 25 µl of ADP (final concentration : 7 µM) was added thereto. The mixture was measured for 5 minutes by using a blood platelet aggregation ability measurement apparatus (Aggricoda TM·PA-3210; made by Kyoto Daiichi Kagaku Co.) to give the maximum aggregation, and the concentration of the test drug to bring 50 % inhibition of the maximum aggregation (IC₅₀) was calculated.

Results are shown in Table 1 wherein the compound number is as defined in the following example as used in the following experiment.

**Table 1**

| Test drug | IC₅₀ value (nM) |
|---|---|
| Compound 3 | 21.5 |

### Experiment 2 [Solubility and Stability Tests]

Solubility and stability tests were carried out according to the method of R.H. Blythe et al [J. Pharm. Sci., vol. 66, page 785 (1977)].

A Britton - Robinson broad buffered solution (pH 4) was prepared, and adjusted to 0.125 of the ionic strength by adding sodium chloride. An excess amount of Compound 3 was added to the resulting solution and dissolved with heating, and then after stirring at 25°C for a desired time, the solubility which became constant was measured by HPLC under the following conditions.
Column : TSK-Gel, ODS-80TM, 4φ × 150 mm
Column temperature : 50°C
Mobile phase : Water : CH₃CN : SDS : phosphoric acid = 500:500:3:1
Detection wave length : 246 nm
Flow rate : 1.0 ml/min.
Injection amount : 10 µl

As a result, the solubility of Compound 3 is 4.3 mg/ml.

A phosphate buffer (pH 7) (0.1M disodium hydrogenphosphate + 0.1M sodium dihydrogenphosphate) was prepared, and adjusted to pH 4.0 with phosphoric acid. Compound 3 was added to the buffered solution at the concentration of 20 µg/ml, and which was used as a test solution. The content of Compound 3 in the test solution stored at 100°C and 65°C respectively was determined, and the rate constant of decomposition at each temperature was calculated. As a result, it was estimated that the term for remaining a 95% content of Compound 3 at 25°C was 17.6 years.

### BEST MODE FOR CARRYING OUT THE INVENTION

The present invention is illustrated in more detail by the following examples.

### Reference Example 1

### N-(2-Methoxycarbonylethyl)-2-[4-(methylthioimidoyl)benzoylimino]-3,4-dimethyl-3H-thiazoline-5-carboxamide methylsulfonate (Compound 1)

A mixture of N-(2-methoxycarbonylethyl)-2-(4-thiocarbamoylbenzoylimino)-3,4-dimethyl-3H-thiazoline-5-carboxamide (104 g), dimethyl sulfate (86.1 ml) and N,N-dimethylformamide (400 ml) was stirred at room temperature for 4 hours. To the reaction solution was added 1.5 ℓ of acetone under ice-cooling, and the resulting crystals were collected by filtration and washed with acetone to give 126.5 g of the title compound.

m.p. 164 - 167°C

### Example 1

### N-(2-Methoxycarbonylethyl)-2-{4-[4-(pyridin-2-yl)piperadinylimidoyl]benzoylimino}-3,4-dimethyl-3H-thiazoline-5-carboxamide [Compound of Formula I wherein R¹ is a methyl group and R² is a methyl group] methylsulfate (Compound 2)

A mixture of Compound 1 (10.0 g), 1-(pyridin-2-yl)piperadine (4.48 g), acetic acid (1.73 ml) and methanol (150 ml) was heated under reflux for 2 hours. After evaporation of the solvent from the reaction mixture, toluene was added, and the resulting crystals were collected by filtration and recrystallized from a mixture of methanol and acetone to give 10.52 g of the title compound.

m.p. 138 - 142°C

### Example 2

### N-(2-Carboxyethyl)-2-{4-[4-(pyridin-2-yl)piperadinylimidoyl]benzoylimino}-3,4-dimethyl-3H-thiazoline-5-carboxamide [Compound of Formula I wherein R¹ is a methyl group and R² is a hydrogen atom] (Compound 3)

A mixture of Compound 2 (14 g), 5 % sodium hydroxide solution (57.4 ml) and isopropyl alcohol (140 ml) was stirred at room temperature for 40 minutes. To the reaction solution was added an aqueous sodium dihydrogenphosphate solution, and the resulting crystals were collected by filtration to give 10.32 g of the title compound.

m.p. 209.5 - 210.5°C

## Claims

1. A thiazoline derivative represented by the formula : 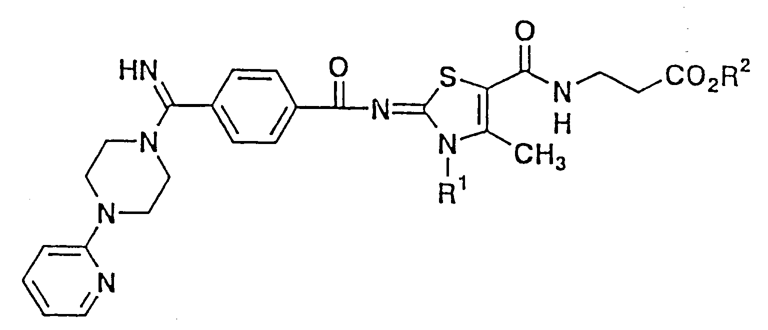 wherein R¹ is an alkyl group having 1 to 4 carbon atoms and R² is a hydrogen atom or an alkyl group having 1 to 4 carbon atoms, or a pharmaceutically acceptable salt thereof.

2. A pharmaceutical composition for inhibition of blood platelet aggregation comprising the thiazoline derivative or the pharmaceutically acceptable salt thereof according to Claim 1 and a pharmaceutical carrier.

3. Use of the thiazoline derivative or the pharmaceutically acceptable salt thereof according to Claim 1 for inhibition of blood platelet aggregation.
